**Europäisches Patentamt**

**European Patent Office**

**Office européen des brevets**

(11) Publication number: **0 131 546**
**B1**

## (12) EUROPEAN PATENT SPECIFICATION

(45) Date of publication of patent specification: **23.01.91**

(21) Application number: **84830146.1**

(22) Date of filing: **10.05.84**

(51) Int. Cl.⁵: **G 01 N 33/531,**
**G 01 N 33/545**

(54) **Method for stably binding antigens and allergens to polystyrene and supports obtained.**

(30) Priority: **13.05.83 IT 2110883**

(43) Date of publication of application:
**16.01.85 Bulletin 85/03**

(45) Publication of the grant of the patent:
**23.01.91 Bulletin 91/04**

(84) Designated Contracting States:
**AT BE CH GB LI LU NL**

(56) References cited:
**EP-A-0 011 030**
**EP-A-0 028 132**
**EP-A-0 139 526**
**WO-A-81/01860**
**FR-A-2 306 997**
**US-A-3 720 760**
**US-A-4 001 583**

**J. Immunol. Methods 95, (1986) 177**
**J. Sci. Food Agric. 47 (1989), 311**
**J. Allergy Clin. Immunol. 59 (1977), 314**

(73) Proprietor: **LABORATORIO FARMACEUTICO**
**LOFARMA s.r.l.**
**Viale Cassala, 40**
**I-20143 Milano (IT)**

(72) Inventor: **Brenna, Oreste Vittore**
**Via Vespucci 11**
**I-20093 Cologno Monzese (Milano) (IT)**
Inventor: **Falagiani, Paolo**
**Via Vallazze 20**
**I-20131 Milano (IT)**

(74) Representative: **de Simone, Domenico et al**
**Ing. Barzanò & Zanardo Roma S.p.A. Via**
**Piemonte 26**
**I-00187 Roma (IT)**

Courier Press, Leamington Spa, England.

# Description

This invention relates to a method for stably binding antigens and allergens to solid supports.

More particularly, this invention relates to a method for stably binding antigens and allergens to polystyrene surfaces, so that said surfaces become suitable to the detection of specific antibodies by means of radioimmunological or immunoenzymatic procedures.

As is well known, it is a common radioimmunological procedure at the present time to use solid phases, which are obtained by binding the antigens to water insoluble substrates in order to make handling easier, for instance the washing operations to remove non-specific antibodies and to measure the bound radioactivity.

According to the Italian patent application No 4568 A/68 of May 22, 1968, said solid phases can be made up of cellulose or Sepharose®, both activated with cyanogen halides, as for example cyanogen bromide.

It is also well known the use of latex particles to that aim.

However, such particles present the drawback of the necessary employment of centrifugation in order to separate the same from their suspension. Thus, solid phases not in the form of particles are preferred, for example nylon, polyethylene, polyvinyl, polystyrene: these solid phases can be available in the form of test pieces, spheres or microplates.

According to US—A—3.646.346, antigens or allergens can be bound to the solid phase through simple surface adsorption.

However, that kind of bond presents some drawbacks, as its stability, dependent upon time and mechanical and termal stresses is not sufficiently reliable, and in addition the amount of antigens or allergens bound, is very small. This small amount of the bound antigen is also a strong drawback, especially when the antigen or the allergen is of a heterogenous composition, as for instance in the case of most of the allergens of pollens, mycophytes and mites. Indeed, in such cases it is necessary to bind an excess of allergen to the solid phase in order to be sure that all allergens of a clinical interest have been represented.

A further drawback which may often occur with that kind of bond is represented by the possibility that a certain solid phase is able to bind the proteins of the serum under test aspecifically, for instance some gammaglobulins. Such a phenomenon, which makes the material unusable in practice, occurs for instance to a high extent with nylon-conjugated allergens, the conjugation occuring by means of glutaraldehyde, said allergens being tested in a RAST system for the detection of the IgE specific antibodies. Moreover, when allergens bound to cellulose by means, for example, of trichloro-S-triazine are used, high values areobtained with positive patients sera, but unacceptable levels of aspecific binding with the negative control sera are also obtained.

A further negative aspect of some binding techniques is the chemical procedure. As is well known, in some procedures a very stable chemical bond, as for example a covalent bond, between the antigen or the allergen and the solid phase is tried.

According to GB—B—1.257.263, glutaraldehyde is a reagent especially suitable for this purpose, said reagent being employed, for instance, at a concentration of 2.5% at pH 6.8, for 12 hours. A drawback of that procedure is the partial chemical modification of antigens and particularly of allergens as a result of exposing the same to the action of glutaraldehyde. Indeed, as is well known, allergens modification by means of glutaraldehyde has been employed for the preparation of the so-called "allergoids", i.e., allergens which have lost to a large extent their ability to react with the IgE class specific antibodies (V. Patterson R. et al., J. Allergy Clin. Immunol. 63, 47, 1979). The so modified allergens can be very useful for therapeutic applications as vaccines, but they cannot be employed for diagnostic purposes.

According to US—A—4 001 583 biological substances are covalently bound to plastic materials whose inside surfaces have been coated with a thin layer of polymerized glutaraldehyde, with or without prior treatment with an aliphatic amine or diamine. In said patent there is stated that the coupling is achieved by means of the self-polymerization of the dialdehyde on the surface of the plastic material, followed by a Schiff base-type coupling of the protein to the active unreacted aldehyde group.

However, it has been found that test materials containing antigens and, more particularly, allergens obtained according to the method disclosed in the above-mentioned patent are not suitable for use in immunoassays for the detection of specific antibodies in the blood, since they give rise to unacceptable levels of aspecific binding.

It is an object of the present invention to provide a method for stably binding antigens or allergens to a solid phase, for instance a polystyrene phase, with no occurrence of the drawbacks pointed out above.

More particularly, it is an object of the present invention to provide a method that allows binding of antigens or allergens to a solid phase, for instance polystyrene, in a way which is stable and resistant to heat and mechanical treatment and that is also specific without causing a complete or a partial denaturation of the same.

Now, it has been found and it is an object of the present invention that these and other objects can be obtained by means of a method which consists of cuusing a polyfunctional aldehyde to react with the substrate solid phase; removing the excess aldehyde by washing; incubating the solid phase so obtained with antigens or allergens, and stabilizing the bond between the aldehyde and the antigens or the allergens by treatment with compounds capable of reducing the iminoaldehy- dic bond, as for instance sodium borohydride, sodium cyanoborohydride or other similar compounds, in which method polystyrene is chosen as the solid support.

As a polyfunctional aldehyde, glutaraldehyde is preferred.

Tests carried out by the Applicant showed that, when operating according to the methods outlined above, denaturation of antigens and of allergens does not occur, because the aldehyde is bound to the solid phase and so it has no possibility of forming intra- and inter-molecular bonds, between different molecules of antigens or allergens. The bond obtained under the conditions of the present meohod is just a covalent bond between the aldehyde and one of the amino-groups of the antigens or allergens. Indeed, it is known that almost all antigens and allergens not of a synthetic origin (for example those derived from pollens, mycophites, mites, food or parasites) are of a proteinous or of a glycoproteinous nature, and so have numerous amino-groups ($—NH_2$) available for that kind of bond.

The solid phases prepared according to the method of the present invention were tested with the RAST system for the detection of the IgE specific antibodies in the sera of allergic patients. Results were obtained which are similar to those obtained with cellulose disks activated with cyanogen bromide, but with the advantage with respect to said cellulose disks of the possibility of carrying out the working steps in an automatic way.

In particular, the aspecific bond, i.e., the bond obtained for instance in normal control sera, was in general satisfactorily low and in some instances it was completely absent.

As regards the polystyrene support to be used, very good results were obtained with solid materials whose surfaces had been treated so as to make them rough. Indeed, it is thus possible to increase the reaction surface and, as a consequence, the amount of antigens or allergens bound. Before the treatment, it is advantageous to wash the material, especially when it has a rough surface, for example the material in the form of spheres, with water or preferably with a dilute saline solution, in order to completely remove the working residual dust which could negatively affect the subsequent operations.

However, smooth polystyrene surfaces can also be employed with satisfactory results, for example test pieces or microplates can be employed. Such smooth surfaces, if worked accurately, can also be suitable for the colorimetric determination of the optical density with suitable devices: that is particularly advantageous for carrying out immunoenzymatic determinations. After the washing operations and before the treatment with aldehyde, the support is dried with an air flow.

In addition to the glutaraldehyde, other polyfunctional aldehydes can be employed having 4—12 carbon atoms.

The concentration of glutaraldehyde or in general of polyfunctional aldehyde is not critical, and it can be chosen within a quite wide interval, for example from 0.01 up to 1 M; concentrations between 0.1 and 0.5 M are preferred.

Glutaraldehyde can be dissolved in a buffer solution, for example a buffer solution of sodium borate or phosphate, at a pH value in the alkaline range, between 7.1 and 9. The reaction time between polystyrene and glutaraldehyde is not critrical and it can be chosen within a very large interval; a time of some hours showed sufficient to give maximum activation. During the reaction it is advisable to keep the reactants stirred. When the reaction with glutaraldehyde is over, the support is repeatedly and accurately washed with a buffer solution of the kind employed in the preparation of the glutaraldehyde solution.

The polystyrene support so washed is then incubated with the antigens or the allergens that are to be covalently bound to the plastic material. The concentration of such antigens or allergens is variable from instance to instance, and it is determined by means of preliminary experiments. In general, the optimum concentration can oscillate between 0.1 µg/ml and 2 mg/ml.

The optimum pH value of the bond forming reaction is usually slightly alkaline and between 7.1 and 9. After a few minutes of incubation, the bond between the antigens or the allergens and the substrate is already surficient for carrying out the next radioimmunological or immunoenzymatic test; however, it is preferred to prolong the bond formation reaction for some hours, or for a whole night. In some cases and with some antigens or allergens, it is advisable to add to their solution an inert protein, for example a bovine albumin, or amino acids, for example lysine or organic amines, for example monoethanolamine; in order to obtain some spacing of the antigen or allergen molecules on the plastic surface so as to improve their steric availability and consequently their immunological reactivity. When the bond formation reaction between the antigens or allergens and the substrate is over, the polystyrene support is repeatedly and accurately washed with a buffer solution to remove the unbound excess reactant. The same buffer solution is preferably employed as the buffer solution for the washing step, in which the glutaraldehyde had been dissolved.

According to the present invention, the bond between glutaraldehyde and the antigens or allergens is stabilized by treatment with sodium borohydride. Such stabilization treatment also prevents possible reaction of serum proteins with still available reactive sites.

Small amounts of sodium borohydride are sufficient for obtaining the desired effect. For example, sodium borohydride concentrations can be employed in the range from 0.1 up to 1 g/l, at a slightly alkaline pH, for example between 7.1 and 8 and for a period of some hours.

After washing the support repeatedly with the same buffer solution as above, the support itself is treated with a buffer solution containing an inert protein, for example human albumin, bovine serum albumin or horse serum albumin. The object of said treatment is that of obtaining a hydrophilic, immunologically inert coating on the plastic material, which coating causes in the next working steps the aspecific binding of the proteins and the serum antibodies to decrease. The solid phases so prepared, containing the allergen or the antigen bound in a stable way, can be stored in a buffer solution as it is or otherwise added with an inert protein and a non ionic surface active agent, for example the surfactant commercially available under the name of Tween® 20 or Tween® 80, or alternatively the solid phases can be stored in the dry state. In the latter case, the drying operation can be performed through lyophilization, or otherwise by means of an air flow, preferably it is performed in any case after a washing step with distilled or deionized water in order to remove any salts and proteins.

The solid phases obtained according to the method of the present invention were found to be very stable over time, both when stored in the wet and in the dry state; stabilities can be obtained for periods of a year and over.

Such solid phases can be employed for detecting antibodies of the IgE, IgG, IgA, or IgM classes, which are specific for antigens or allergens. The analytical determinations can be applied to human sera or to the sera of domestic animals, either to diagnose an allergic illness or a contagious disease in progress, or to check an immunity state, for example after prophylactic vaccinations.

For a better understanding of the present invention and to show the practical aspect of the same, some examples are given in the following for illustrative purposes.

In the following examples, all parts are given as parts by weight unless expressed differently.

Example I
Preparation of solid phases for the analytical determination by the RAST system of IgE antibodies specific for the allergens of the graminaceous pollens.

300 spheres of rough-surface polystyrene material, diameter 6.4 mm, are washed for 24 hrs. with a NaCl 0.1 M solution in a large amount, then with deionized water and dried in a flow of air. The spheres are than transferred into a tightly-sealed container and treated for 5 hrs. with 200 ml of 0.2 M glutaraldehyde diluted in a buffer solution of trisodium phosphate at pH 7.2 (0.15 M) under continuous shaking by turning the container upside down. The glutaraldehyde solution is then removed and the spheres are washed three times with the same buffer solution of trisodium phosphate. The excess liquid is sucked off and 200 ml is added of a graminaceous allergen containing 0.4 mg of proteins/ml dissolved in a buffer solution of phosphate at pH 7.2, determined according to the Lowry procedure, then the whole mass is stirred for 16 hrs. The spheres are washed with the buffer solution three times, replacing the amount of solution employed with a fresh amount each time. The excess liquid is sucked off and 400 ml is added of a 0.37% solution by weight of sodium borohydride; the whole mass is left to stand for 1 hr. The spheres are washed twice with the buffer solution described above, replacing each time the amount employed with a fresh amount, then the spheres are resuspended in the same buffer solution containing 0.2% bovine albumin and 0.4% Tween 20 (registered Trade Mark), and they are stored at +4°C. When the spheres are used, they are distributed in test tubes of 12 mm diameter, and incubated first with the serum of the patient under test and afterwards with an anti-IgE antibody, such antibody being [125]I labelled, following the RAST procedure which is already known and embodied with solid phases of cyanogen bromide activated cellulose, as described by Falagiani P. et al., Folia Allergol. Immunol. Clin. 25, 579 (1978). After performing the operations according to the RAST procedure, the radioactivity of the test tubes, i.e., of the spheres contained in the same is determined by a gamma-counting device, and the results obtained are processed according to the criteria given in the paper quoted above. The same procedured can be employed fov the determination of the IgE antibodies specific for other allergens, for example the allergens from mites, mycophytes, pollens, food and parasites.

The antigen concentration can be modified on the basis of the information obtained in the course of previous experiments.

Example II
Preparation of a solid phase made up of *Micropolispora faeni* antigen bound to polystyrene microplates for the determination of specific IgE antibodies by an immunoenzymatic procedure.

A rigid polystyrene microplate is employed of the PBI (Pool Bionalysis Italiana, Milan) "Elisa" type, in which plate 96 U wells are obtained by stamping. 0.3 ml are introduced into each well by a pipette of a 3% glutaraldehyde solution, equivalent to a 0.3 M solution, diluted in a pH 8 buffer solution of phosphate, and the wells are incubated for 4 hrs. with slight stirring. The solution is removed by suction with a vacuum pump, and the wells are filled and successively emptied by turning them upside down five times with the borate buffer solution. The buffer solution is removed and 0.3 ml of *Micropolispora faeni* antigen is pipetted into the wells, said antigen solution having a protein strength of 1.1 mg/ml. The plates are covered with a Parafilm® sheet and the whole is kept under slight stirring for 18 hrs. The antigen is removed by suction and the microplate is washed by five repeated filling and emptying operations with the buffer solution described above.

The plate is emptied and the wells are filled with 0.4 ml of a 0.5% solution of sodium borohydride in a buffer solution of trisodium phosphate at pH 7.2, then the wells are left to stand for 2 hrs.

The microplate is washed with the trisodium phosphate buffer solution and lastly with the same buffer solution containing 0.2% bovine albumin.

Plates can be stored till they are used, at a temperature between +4°C and −20°C, or they are rinsed with deionized water, then left to dry and stored at room temperature. Such solid phases can be employed for carrying out the analytical determination of IgG's by means of an immunoenzymatic procedure as described by Voller A. et al. in "Handbook of Clinical Immunology", N. R. Rose and H. Friedman, eds., p. 506, American Society for Microbiology, 1976. 200 µl of serum, at properly scaled down concentrations, is pipetted into each well, then the wells are incubated for two hrs., washed three times with the trisodium phosphate buffer solution described above, containing 0.4% Tween® 20. 200 µl of anti-IgG antibody conjugated with peroxidase is added, then it is incubated overnight at +4°C, washed three times with the phosphate buffer solution containing 0.4% Tween 20 (registered Trade Mark). 200 µl is added of a solution containing 34 mg of ortho-phenylendiamine in 100 ml of a citrate-posphate buffer solution at pH 5.0. The solution is left in the dark for 30 minutes, then the reaction is stopped with 50 ml of sulfuric acid at 12.5% wt/volume, and the developed color strength is determined with a suitable colorimeter at a wavelength of 492 mm.

The microplate prepared as above can be employed also for the determination of protein A of *Staphylococcus aureus* in solution, peroxidase labeling being employed in this case as well. The enzyme peroxidase can also be replaced with alkaline phosphatase or with beta-galactosidase.

If separated single wells of PBI type polystyrene or "Indiwell cells" are employed instead of using the microplates described above, radioimmunological determinations can be performed in a similar procedure. In that case, after the incubation with the anit-IgG antibody or with the protein A of the *Staphylococcus aureus,* both labeled for example with [125]I, and the washing steps already described, said wells will be inserted into test tubes and their radioactivity will be determined with a gamma-counter.

## Claims

1. Method for stably binding antigens and allergens to solid supports, wherein the solid support is first treated with a polyfunctional aldehyde, then the excess polyfunctional aldehyde is removed by washing, the resulting solid phase is incubated with the antigens or the allergens and the bond between the aldehyde and the antigens or the allergens is stabilized by means of treatment with a reducing agent, characterized in that said solid support is made of polystyrene, and said treatment with a polyfunctional aldehyde is carried out at a pH between 7.1 and 9 and for a total reaction time of some hours, thereby resulting in binding of said polyfunctional aldehyde on to said polystyrene surface by hydrophobic adsorption.

2. A method according to claim 1, in which said reducing agent is sodium borohydride or sodium cyanoborohydride.

3. A method according to claim 1 or 2, in which the polyfunctional aldehyde is glutaraldehyde.

4. A method according to claim 1 or 2, in which the polyfunctional aldehyde has from 4 up to 12 carbon atoms.

5. A method according to any of the preceding claims, wherein the solid support is in the form of spheres or of microplates.

6. A method according to any of the preceding claims, wherein the concentration of the polyfunctional aldehyde ranges from 0.01 M to 1 M, and preferably it is between 0.1 and 0.5 M.

7. A method according to claim 6, wherein the polyfunctional aldehyde is dissolved in a sodium borate or sodium phosphate buffer solution at a pH between 7.1 and 9.

8. A method according to any of the preceding claims, wherein the excess polyfunctional aldehyde is removed after reaction by washing with the same buffer solution as that employed for the preparation of the aldehyde solution.

9. A method according to any of the preceding claims, wherein an inert protein, amino acids or organic amines are added to the solution containing the antigens or allergens.

10. A method according to any of the preceding claims, wherein the stabilizing treatment is carried out using a sodium borohydride solution that has a concentration between 0.1 and 1 g/l, a pH value between 7.1 and 8, and for a period of some hours.

11. A method according to any of the preceding claims, wherein said support is successively treated with a buffer solution containing an inert protein such as human albumin, bovine serum albumin or horse serum albumin.

12. The use of the test material obtained by the method of any one of the preceding claims for the detection *in vitro* in body fluid samples of the IgE, IgG, IgA or IgM classes antibodies specific for antigens or allergens either to diagnose an allergic or an infectious disease, or to check an immunity state.

## Patentansprüche

1. Verfahren zum stabilen Binden von Antigenen und Allergenen an feste Träger, worin der feste Träger zuerst mit einem polyfunktionellen Aldehyde behandelt wird, dann der überschüssige polyfunktionelle Aldehyd durch Waschen entfernt wird, die resultierende Festphase mit den Antigenen oder den Allergenen inkubiert wird und die Bindung zwischen dem Aldehyd und den Antigenen oder den Allergenen mittels Behandlung mit einem Reduktionsmittel stabilisiert wird, dadurch gekennzeichnet, daß der feste Träger aus Polystyrol hergestellt ist und die Behandlung mit einem polyfunktionellen Aldehyd bei einem pH zwischen 7,1 und 9 und einer Gesamt-Reaktionsdauer von einigen Stunden durchgeführt wird, so daß dadurch eine Bindung des polyfunktionellen Aldehyds an die Polystyrol-Oberfläche durch hydrophobe Adsorption resultiert.

2. Verfahren nach Anspruch 1, worin das Reduktionsmittel Natriumborhydrid oder Natriumcyanoborhydrid ist.

3. Verfahren nach Anspruch 1 oder 2, worin der polyfunktionelle Aldehyd Glutaraldehyd ist.

4. Verfahren nach Anspruch 1 oder 2, worin der polyfunktionelle Aldehyd von 4 bis 12 Kohlenstoffatome besitzt.

5. Verfahren nach einem der vorhergehenden Ansprüche, worin der feste Träger in Form von Kugeln oder Mikroplatten ist.

6. Verfahren nach einem der vorhergehenden Ansprüche, worin die Konzentration des polyfunktionellen Aldehyds von 0,01 M bis 1 M reicht und vorzugsweise zwischen 0,1 und 0,5 M ist.

7. Verfahren nach Anspruch 6, worin der polyfunktionelle Aldehyd in einer Natriumborat- oder Natriumphosphat-Pufferlösung bei einem pH zwischen 7,1 und 9 gelöst wird.

8. Verfahren nach einem der vorhergehenden Ansprüche, worin der überschüssige polyfunktionelle Aldehyd nach Reaktion durch Waschen mit derselben Pufferlösung entfernt wird, die für die Herstellung der Aldehyd-Lösung verwendet wurde.

9. Verfahren nach einem der vorhergehenden Ansprüche, worin ein inertes Protein, Aminosäuren oder organische Amine zur Lösung gegeben werden, welche die Antigene oder Allergene enthält.

10. Verfahren nach einem der vorhergehenden Ansprüche, worin die Stabilisierungs-Behandlung durchgeführt wird, indem man eine Natriumborhydrid-Lösung verwendet, die ein Konzentration zwischen 0,1 und 1 g/l, einen pH-Wert zwischen 7,1 und 8 besitzt, und für die Dauer von einigen Stunden.

11. Verfahren nach einem der vorhergehenden Ansprüche, worin der Träger sukzessiv mit einer Pufferlösung behandelt wird, die ein inertes Protein wie etwa humanes Albumin, Rinderserumalbumin oder Pferdeserumalbumin enthält.

12. Verwendung des Testmaterials, das durch ein Verfahren nach einem der vorhergehenden Ansprüche erhalten wurde, für die Bestimmung in vitro von Antikörpern der Klassen IgE, IgG, IgA oder IgM, die spezifisch für Antigene oder Allergene sind, in Proben von Körperflüssigkeiten, entweder zur Diagnose einer allergischen oder infektiösen Krankheit oder zum Überprüfen eines Immunstatus.

**Revendications**

1. Méthode pour attacher stablement antigènes et allergènes à des supports solides, dans laquelle le support solide est au début traité avec un aldéhyde polyfonctionnel, ensuite l'excés du aldéhyde polyfonctionnel est enlevé au moyen de lavage, la phase solide en résultant est incubée avec les antigènes ou allergénes et la liaison entre l'aldéhyde et les antigènes ou allergènes est stabilisée par traitement avec un agent réducteur, characterisée en ce que ledit support solide est fait en polystyrène et ledit traitement avec un aldéhyde polyfonctionnel est réalisé à un valeur de pH entre 7,1 et 9 et pendant le temps de réaction total de quelques heures, de façon à obtenir la liaison du dit aldéhyde polyfonctionnel avec la surface du dit polystyrène par une adsorption hydrophobe.

2. Méthode selon la revendication 1, dans laquelle ledit agent réducteur est sodium borohydrure ou sodium cyanoborhydrure.

3. Méthode selon les revendications 1 ou 2, dans laquelle l'aldéhyde polyfonctionnel est glutaraldéhyde.

4. Méthode selon les revendications 1 ou 2, dans laquelle l'aldéhyde polyfonctionnel a de 4 jusqu'à 12 atomes de carbone.

5. Méthode selon les revendications précédentes, dans laquelle le support solide est en forme de sphères ou microplaques.

6. Méthode selon une des revendicatuons précédents, dans laquelle la concentration du aldéhyde polyfonctionnel est 0,01 M jusqu'à 1 M et préférablement entre 0,1 M et 0,5 M.

7. Méthode selon la revendication 6, dans laquelle l'aldéhyde polyfonctionnel est dissous dans une solution tampon à un valeur de pH entre 7,1 et 9.

8. Méthode selon une quelconque des revendications précédentes, dans laquelle l'excés du aldéhyde polyfonctionnel est enlevé au moyen de lavage après la réaction avec la même solution tampon employée pour la préparation de la solution d'aldéhyde.

9. Méthode selon une quelconque des revendications précédentes, dans laquelle une protéine inerte, des aminoacides ou des acides organiques sons ajointés à la solution contenante les antigènes ou allergènes.

10. Méthode selon une quelconque des revendications précédentes, dans laquelle le traitement de stabilisation est réalisé au moyen d'une solution de borohydrure ayant une concentration entre 0,1 et 1 g/l, un valveur de pH entre 7,1 et 8 et dans une période de quelques heures.

11. Méthode selon une quelconque des revendications précédentes, dans laquelle ledit support est traité successivement avec une solution tampon contenant un protéine inerte, tel que une albumine humaine, sérumalbumine bovine ou chevaline.

12. Usage des matériaux de test obtenus par la methode d'une quelconque des revendications précédentes pour détecter in vitro dans des échantillons dés fluides corporels les anticorps des classes IgE, IgG, IgA ou IgM spécifiques pour les antigènes ou l'allergènes, afin de diagnostiguer une maladie allergique ou une maladie infectieuse ou afin de contrôler un état d'immunité.